(19) Europäisches Patentamt
European Patent Office
Office européen des brevets

(11) Publication number: **0 001 162**
**B1**

(12) # EUROPEAN PATENT SPECIFICATION

(45) Date of publication of patent specification: **15.12.82**

(51) Int. Cl.³: **A 61 K 7/32, A 61 K 7/38**

(21) Application number: **78300316.3**

(22) Date of filing: **23.08.78**

(54) **Antiperspirant compositions for topical uses.**

(30) Priority: **26.08.77 GB 3598177**

(43) Date of publication of application:
**21.03.79 Bulletin 79/6**

(45) Publication of the grant of the patent:
**15.12.82 Bulletin 82/50**

(84) Designated Contracting States:
**DE FR GB NL SE**

(56) References cited:
DE - A - 1 617 337
DE - A - 2 263 509
DE - A - 2 304 156
FR - A - 1 322 144
FR - A - 2 121 690
FR - A - 2 250 516
FR - A - 2 263 742
GB - A - 23 863
GB - A - 425 059
GB - A - 1 482 756
US - A - 3 172 817

(73) Proprietor: **UNILEVER PLC**
**Unilever House Blackfriars P O Box 68**
**London EC4P 4BQ (GB)**
(84) **GB**
(73) Proprietor: **UNILEVER NV**
**Burgemeester s'Jacobplein 1 P.O. Box 760**
**NL-3000 DK Rotterdam (NL)**
(84) **DE FR NL SE**

(72) Inventor: **Chaudhuri, Dwaipayan**
**7 Chester Close**
**Ashford Middlesex (GB)**
Inventor: **Stebles, Malcolm Richard Douglas**
**18 Keble Road**
**Maidenhead Berkshire (GB)**

(74) Representative: **Tonge, Robert James et al,**
**Unilever PLC Patent Division PO Box 31**
**London EC4 4AN (GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Anti perspirant compositions for topical uses

The invention relates to antiperspirant compositions for topical application to human skin. More particularly the invention relates to antiperspirant compositions which can be dispensed from valved aerosol containers or pump spray dispensers or roll ball applicators to be deposited onto the skin in particulate form, or as a film or layer. These compositions are based on moisture-absorbent materials which are generally non-astringent in nature and which help to make the composition substantially non-staining and milder to the skin.

Conventional antiperspirant compositions traditionally contain an astringent, such as aluminium chlorhydrate, zirconium oxychloride aluminium hydroxychloride complex or zinc phenol sulphonate, which is believed to depress the formation of perspiration from sweat glands. These astringents are commonly applied to the skin in the form of an aerosol spray.

In our French Patent Applications Nos. 2,263,742 and 2,250,516 there is described an entirely new type of composition for limiting the manifestation of perspiration on the skin which does not necessarily require the use of astringent perspiration depressants, such as those exemplified above, and which can therefore reduce the irritating effect that astringents and their by-products sometimes produce. Therefore, instead of relying entirely on chemical suppression of perspiration at source, we employ a means whereby perspiration can be absorbed at the skin surface as soon as it is formed, thus maintaining the skin in an apparently dry condition. This is achieved by applying to the skin a composition containing a special polymer having a high capacity for absorbing superficial skin moisture, together with a substance which controls the moisture absorbing capacity of the polymer, which improves its adhesion to the skin and which enhance its superficial tactile properties. It follows that transfer of perspiration from the skin to adjacent clothing can also be limited or prevented completely by this means which is at least as effective as conventional astringent antiperspirant materials, but which does not suffer from the disadvantages associated with such materials.

Extensive laboratory investigations and in vivo trials involving human subjects have been directed to the problems of controlling the degree to which the polymer absorbs water and of enhancing the substantivity of the polymer to the skin and its superficial tactile properties.

Early investigations showed, for example, that although a polymer might intrinsically be capable of absorbing an amount of water equal to many times its own weight, the swelling of the polymer on the skin as it absorbed perspiration caused it to become obtrusive to the extent that the subject expressed a dislike of the 'feel' of the swollen polymer on the skin.

It was also observed that a film or layer of the polymer when applied to the skin lacked sufficient substantivity to remain intact on the skin over an extended period of time, for example during a working day. Body movement and contact by clothing adjacent to the treated area of skin tended to abrade the film or layer or polymer such that it would roll-up or flake-off the skin with consequent loss of antiperspirancy.

It was furthermore noted by human subjects that the 'feel' of the film or layer of the polymer lacked the desirable smoothness which was necessary if the presence of the polymer on the skin was to remain unobtrusive. A slight roughness of the film or layer of polymer would, for example, tend to 'catch' on adjacent clothing and skin: this was considered to be an undesirable attribute.

The problems of how to control moisture uptake, how to improve substantivity to the skin and how to improve 'feel' have now been solved, according to the invention, by presenting the composition in a two-phase form such that the polymer (solid phase) is suspended in a solvent or propellant (liquid phase) and by including in the liquid phase a particular surfactant having a melting point within the range of from 30 to 75°C.

French Patent Application No. 2,263,742, mentioned above, discloses various types of antiperspirant composition based on an adsorbent polymer, for example, liquids and semisolids, dry powders and waxy sticks. it is suggested that when the composition is in the form of a solid such as a waxy stick, a conventional carrier such as stearic acid may be used, but this suggestion is made only in connexion with solid antiperspirant compositions. The use of sodium stearate as a thickener is also disclosed, but this material is highly water-soluble and has a softening point of 205°C.

Furthermore, both this document and French Patent Application No. 2,250,516, also mentioned above, disclose the possible use of poly (lower-alkoxylated) cetyl alcohols as carriers. These materials, details of which are given in British Patent Specification No. 1,445,437 (Colgate-Palmolive Company) are highly water-soluble and are specifically known to be perspiration-soluble.

British Patent Specification No. 1,482,756 (Jeyes) discloses an antiperspirant composition based on a highly moisture-absorbent gel-forming polymer suspended in a liquefied aerosol propellant. The composition may contain as suspending or emulsifying agent a surface-active agent such as sorbitan monooleate, which has a melting point substantially lower than 30°C. The inclusion of ethoxylated lanolin derivatives as emollients is also suggested, but the only specific material mentioned is "Solulan (Trade Mark) 97" which according to the trade

literature of its manufacturer Amerchol is a liquid at ambient temperature and is water-soluble.

In precise terms, the present invention provides an antiperspirant composition having a solid phase and a liquid phase, the solid phase comprising a moisture-absorbent, water-insoluble polymer and the liquid phase comprising an organic solvent in which the said polymer is substantially insoluble and a surfactant, characterised in that

(i) the polymer amounts to from 0.1 to 30% by weight of the composition, is substantially dry to the touch when swollen with water and has a water-absorption value of at least 2,
(ii) the surfactant is selected from cetyl alcohol, isocetyl alcohol, stearyl alcohol, stearic acid, palmitic acid, glycerol monolaurate, and mixtures thereof, and amounts to from 0.1 to 15% by weight of the composition, and
(iii) the organic solvent amounts to from 1 to 99.8% by weight of the composition.

The surfactant has a melting point within the range of from 30 to 75°C.

The invention also provides a process for preparing an antiperspirant composition which comprises mixing together the moisture-absorbent water-insoluble polymer, the surfactant and the organic solvent as herein defined.

The invention also provides a method for absorbing perspiration which comprises applying topically to the skin, particularly in the region of apocrine sweat glands, an antiperspirant composition as therein defined.

The moisture-absorbent water-insoluble polymer will generally have a water-absorption value of at least two, preferably from 5 to 500 and most preferably from 10 to 50.

The water absorption value of a polymer is measured by spraying it in a finely divided dry state onto a tared filter paper using an anhydrous propellant as a vehicle. After allowing the propellant to evaporate, the filter paper is weighed again to give the dry weight of the polymer which has been deposited.

Water is then sprayed onto the reverse side of the filter paper until the deposited polymer is just wet to the touch and also appears wet when observing the reflectance of a beam of light directed onto the top surface of the polymer layer; the water is metered as it is sprayed on so tnat the amount used to reach this endpoint is known. The paper is weighed again to give the 'wet' weight of the polymer and the filter paper and hence the amount of water absorbed by the polymer and the filter paper. The same amount of water is then sprayed onto a second tared filter paper without polymer, and the amount of water absorbed by the paper determined by weighing. This enables the proportion of absorbed water taken up by the paper in the original experiment to be calculated, and hence the water taken up by the polymer alone.

The water absorption value is given by the weight of water absorbed by the polymer divided by the dry weight of the polymer.

The polymer is preferably chosen from one or more of the following polymers:

a) a cross-linked starch
b) other anionic polyelectrolytes, and
c) a base hydrolysed starch/polyacrylonitrile graft copolymer.

More specifically, an example of the cross-linked starch is a substantially water-insoluble cross-linked gelatinised starch, in which the degree of substitution of the cross-linking groups is 0.001 to 0.04, which is preferably, but not necessarily, substituted by ionic groups attached to the starch by ether linkages, these groups, when present, being associated with mono- or di-valent counterions.

Although the degree of substitution of the cross-linking groups can be relatively low, the cross-linked starch is substantially insoluble in water.

The cross-linking of the starch molecules may be effected by ether bridges of the formula —O—R—O—, where R is an aliphatic group, which may be substituted by one or more hydroxy groups, containing 1 to 10 carbon atoms. Preferably R is $CH_2CH(OH)CH_2$—, which is the case when the starch is cross-linked using epichlorhydrin as cross-linking agent.

The ionic groups, when present, preferably have the formula Z—$R^1$ and $R^1$ is an alkylene group having 1 to 5 carbon atoms and Z is an anionic group selected from carboxyl, sulphonic or phosphonic groups or a cationic group of the formula:

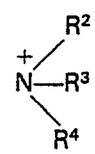

where $R^2$ is hydrogen or lower alkyl, and $R^3$ and $R^4$ are lower alkyl or are alkylene groups linked together to form a five or six-membered heterocyclic ring. Particularly suitable materials are those wherein $R^1$ is an alkylene group containing 1 or 2 carbon atoms and Z is —$COO^-$ and preferred materials are carboxymethylated cross-linked gelatinised starches.

When the polymer is substituted by ionic groups, the degree of substitution of the ionic groups will generally be at least 0.1 and is desirably at least 0.2 to obtain the preferred higher water absorption values.

When Z is an anionic group, the counterion preferably is an alkali metal, alkaline earth metal, ammonium or substituted ammonium ion. The substituted ammonium derivatives may be those in which one or more hydrogen atoms are replaced by $C_{1-4}$ alkyl or $C_{2-4}$ hydroxyalkyl groups or in which the nitrogen atom forms part of a heterocyclic ring. An example of such a

substituted ammonium ion is tetramethylammonium. Preferred counter-ions when Z is an anionic group are the sodium, potassium and ammonium ions. When Z is a cationic group, the counterion may be, for example, chloride, bromide or sulphate.

Particularly preferred absorbent materials of this invention are the sodium and ammonium salts of carboxymethylated epichlorhydrin cross-linked gelatinised starch having a water absorption value of at least 5 and being insoluble in water to the extent of at least 99% by weight. The sodium or ammonium salts are hereinafter referred to in the examples as "sodium cross-linked starch" or "ammonium cross-linked starch" respectively.

Cross-linked gelatinised starch can be prepared by the process described in our British Patent Application No. 2118/77.

Examples of the anionic polyelectrolyte, other than the cross-linked starches as hereinbefore defined, which are alternative polymers having a water absorption value of at least 2 are water-insoluble ionic complexes of a water-soluble anionic polyelectrolyte and a polyvalent metal cation having a valency of at least three, the cation being present in the amount of 0.01 to 5.0 milli-equivalents per gram of polyelectrolyte.

The polyelectrolyte generally contains anionic groups, such as carboxylate, sulphonate, sulphate and phosphate groups or mixtures thereof. Preferably the polyelectrolyte is polyacrylic acid.

The cation is a transition metal, preferably aluminium, iron, chromium, zirconium, titanium or mixtures thereof. The cation is preferably present in the amount of 0.1 to 1.0 milliequivalents per gram of polyelectrolyte.

A particularly preferred polyelectrolyte is polyacrylic acid having from 40 to 85% of its carboxylate groups neutralised, the cation being aluminium which is present in the amount of from 0.1 to 1.0 milliequivalents per gram of polyelectrolyte.

These polyelectrolytes and their preparation are further described in German Patent Application (DTOLS) No. 2 609 144 (National Starch and Chemical Corporation).

Further examples of the anionic polyelectrolyte are water-insoluble covalently cross-linked anionic polyelectrolytes.

The anionic polyelectrolytes can be water-soluble polyelectrolytes which are covalently cross-linked to render them insoluble yet capable of absorbing water.

Examples of these water-soluble anionic polyelectrolytes to be covalently cross-linked are natural polymers, such as anionic derivatives of starch and cellulose, and synthetic polymers such as carboxylic homopolymers or copolymers containing at least 20 mole per cent carboxylic acid units such as polyacrylic acid.

Examples of the covalent cross-linking compounds which can be copolymerised with the polyelectrolytes are divinyl compounds, such as divinyl benzene, divinyl diethylene glycol diether, divinyl diphenyl silicone and divinyl sulphone; allyl compounds, such as triallyl cyanurate, trimethylol propane diallyl ether, allyl methacrylate, allyl acrylate, allyl crotonate, diallylphthalate, diallyl succinate and diallyl sucrose; polyfunctional acrylates and methacrylates, such as tetraethylene glycol diacrylate, triethylene glycol dimethacrylate, pentaerythritol tetra acrylate, ethylidene dimethylacrylate and trimethylol propane trimethacrylate; and polyfunctional arylamides and methacrylamides, such as N,N'-methylene bis-acrylamide and N,N'-methylene bismethacrylamide.

These water-insoluble covalently cross-linked anionic polyelectrolytes and their preparation are further described in Netherlands Patent Application No. 7 604 518 (National Starch and Chemical Corporation).

Any of the water-insoluble anionic polyelectrolyte complexes as herein described can be surface treated with a polyvalent metal cation to maximise absorption by the polymer particles of moisture.

The preferred polyvalent metal cations for surface treatment are aluminium, zirconium, chromium, titanium and zinc.

These surface-treated water-insoluble anionic polyelectrolyte complexes and their preparation are also further described in Netherlands Patent Application No. 7 604 518 (National Starch and Chemical Corporation).

An example of a surface treated anionic polyelectrolyte is National Starch Resyn® 78—3803 which is potassium polyacrylate cross-linked with aluminium.

Examples of the base hydrolysed starch polyacrylonitrile graft copolymer are those comprising water-insoluble alkali metal salts of saponified gelatinised starch and saponified polyacrylonitrile in mole ratios of from 1:1.5 to 1:9. Copolymers such as these are identified and prepared by the methods set out in Journal of Applied Polymer Science, Volume 13, pages 2007—2017 (1969), and Volume, 15, pages 3015—3024 (1971).

The moisture absorbent polymer should be in the form of a finely-divided powder having a particle size not greater than 100 $\mu$, preferably not greater than 60 $\mu$.

The quantity of the moisture absorbent polymer to be employed is generally at least 0.1, usually from 0.1 to 30% by weight of the composition, but the actual amount chosen will depend on the nature of that composition and the water absorption value of the polymer. Preferably, the amount of polymer is from 0.5 to 20%, most preferably from 1 to 15% by weight of the composition.

It is apparent that compositions containing less than 0.1% by weight of the composition of polymer are unlikely to perform effectively as

antiperspirants, whereas where the polymer forms more than 30% by weight of the composition, the composition may be difficult to dispense or the amount of polymer deposited on the skin when the composition is used as an antiperspirant may be excessive leading to poor consumer acceptance.

The composition also comprises a surfactant having a melting point of from 30° to 75°C, which is selected from cetyl alcohol, isocetyl alcohol, stearyl alcohol, stearic acid, palmitic acid, glycerol monolaurate, and mixtures thereof.

It is important that the chosen surfactant has a melting point within the defined range, since it is the surfactant which influences the ability of the polymer to absorb moisture, improves its substantivity to the skin and enchances the feel of the polymer on the skin. It has for example been shown that when the surfactant is one which has a melting point of less than 30°C, it adversely affects the behaviour of the polymer on the skin in three ways. Firstly, the particles of the polymer in contact with the skin can, under certain circumstances, agglomerate; secondly, the substantivity of the polymer on the skin can be poor, and thirdly, moisture uptake by the polymer on the skin can lack the necessary control to optimise both moisture uptake and swelling of the polymer.

It should be explained that agglomeration can occur in the presence of a low melting point surfactant in the following manner. The particles of polymer when first applied to the skin will usually form a smooth firm, but as they absorb moisture, for example from perspiration, the particles will generally tend to migrate towards each other on the skin surface to form adhering groups of particles known as agglomerates. This is apparently due to the inability of the low melting point surfactant to hold the particles in place on the skin. As perspiration evaporates, the polymer particles revert to the dry state, but because of agglomeration a rough 'sand paper' texture results. The polymer now forms an abrasive textured layer on the skin which is unacceptable to the consumer.

It should also be explained that the agglomerated polymer exhibits poor substantivity to the skin, because the agglomerates are heavier than the individual particles of the polymer and hence are more easily dislodged from the skin.

It should also be explained that when the surfactant has a melting point of less than 30°C, it is unable to reduce moisture uptake from the skin by particles of a polymer having a high water absorption value. It is therefore likely that excessive swelling of the polymer will lead to the formation of an obtrusive layer on the skin which is unacceptable because the presence of the film can then be felt by the user.

It has also been shown that if the melting point of the surfactant exceeds 75°C, it can coat the polymer particles on the skin to such an extent that moisture uptake by the polymer is severely hampered, with the result that there can be inadequate control of perspiration.

It follows that the choice of a surfactant which has a melting point of from 30 to 75°C restricts mobility of the polymer particles on the skin as moisture is absorbed and maintains adequate adherences of the particles to the skin without excessively restricting their ability to absorb moisture from the skin.

The quantity of surfactant to be employed is generally from 0.1 to 15%, preferably from 0.1 to 10% by weight of the composition.

Compositions containing less than 0.1% by weight of a surfactant will tend to be less substantive to the skin than is desirable and will also lack the sufficient control of moisture uptake and so can lead to a film or layer of polymer on the skin which is too obtrusive on absorption of moisture. Compositions containing more than 15% by weight of a surfactant can contribute excessively to the apparent greasiness of the film or layer of polymer on the skin and can severely restrict absorption of moisture with consequent loss of effectiveness of the antiperspirant composition.

The composition also comprises an organic solvent in which the polymer is substantially insoluble. The function of the solvent is to provide the composition with sufficient mobility to distribute the polymer conveniently and uniformly on the skin. The solvent should, however, be capable of subsequently evaporating from the skin within a few minutes, so that the composition is then dry to the touch, without residual 'wetness' or 'greasiness' derived from the solvent. The solvent should accordingly have a boiling point no higher than 150°C, preferably no higher than 100°C.

Examples of solvents are ethyl alcohol, methylene chloride, isopropyl alcohol, ethylene glycol monomethyl ether, low boiling liquid paraffins, such as pentane, hexane, heptane and octane, volatile silicones such as linear and cyclic polydimethyl siloxanes. The solvent can also be a propellant such as trichlorofluoromethane, dichlorodifluoromethane, dichlorotrifluoroethane, monochlorodifluoromethane, trichlorotrifluoroethane, propane, butane, isobutane, dimethylether and carbon dioxide.

The solvent will generally comprise at least 1%, preferably from 55 to 99.8% by weight of the composition, the amount actually selected generally forming the balance of the composition after accounting for the polymer, the surfactant and any optional ingredients that may also be included in the formulation.

The composition can also optionally comprise an emollient which, if present, is preferably a non-hydroxylic emollient, although certain hydroxylic emollients can be employed. The emollient will generally have a melting point of less than 20°C.

Preferred examples of non-hydroxylic emollients are isopropylmyristate, isopropyl

palmitate and dibutylphthalate. Preferred examples of hydroxylic emollients are 2-ethyl-1,3-hexane diol and hexylene glycol.

The quantity of emollient when included in the composition is generally up to 30%, preferably from 5 to 20%, most preferably from 8 to 10% by weight of the composition.

The composition can also optionally comprise a suspending agent which, if present, is one which is hydrophobically treated, for example, hydrophobically treated montmorillonite clays.

It should be explained that montmorillonite clays are clays that contain the mineral montmorillonite, and are characterised by having an expanded lattice. An example is bentonite.

Bentonite is colloidal hydrated aluminium silicate obtained from montmorillonite and has the formula $Al_2O_34SiO_2.H_2O$. A more detailed discussion of bentonites can be found in the Kirk-Othmer Encyclopedia of Chemical Technology, 2nd Edition, Vol. 3, (1964) pp 339—360, published by Interscience Publishers.

Examples of hydrophobically treated bentonites are BENTONE® 27 which has the structure

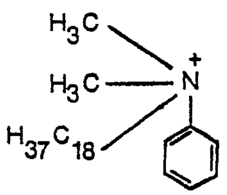

$Cl^-$ hectorite

and BENTONE (Trade Mark) 38 which has the structure

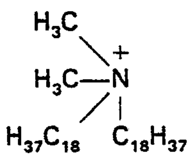

$Cl^-$ hectorite

BENTONES (Trade Mark) are available from the National Lead Company. Montmorillonite clays can also be prepared synthetically and can be treated with long chain amines to render them hydrophobic. An example is the hydrophobic form of LAPONITE (Trade Mark) available from Laporte Industries.

Further examples of suspending agents are Acrawax® C (an ethylene diamine condensate with 2 mols stearic acid), zinc ricinoleate, zinc stearate and Aerosil 200 (a pyrogenic silica) and sodium stearate.

The quantity of suspending agents that can optionally be employed is generally up to 8% by weight of the composition.

The composition can also optionally comprise a deodorant. Examples of deodorants are sodium carbonate, sodium bicarbonate, aluminium chlorhydrate, aluminium sulphate and zinc ricinoleate. It will be appreciated that astringent aluminium salts such as the sulphate and chlorhydrate will, if present, also function as antiperspirants.

The deodorant, if present, can comprise from 0.1 to 20% by weight of the composition.

The antiperspirant composition can take the form of a propellant based aerosol product or it can be employed in the absence of a propellant in a pump spray dispenser. Alternatively, the antiperspirant composition can be a lotion containing the polymer suspended or dispersed therein for direct application to the skin with a swab or with the use of a roll-ball applicator.

The invention accordingly also comprises an applicator or other container containing the antiperspirant composition as herein defined.

Compositions of the invention can be prepared by mixing together the polymer, the surfactant and the solvent and optional ingredients to provide a homogeneous dispersion of the solid particulate polymer in a liquid phase.

According to a preferred process, when the solvent comprises an aerosol propellant and a suspending agent is included, the suspending agent is first sheared with the propellant and then the mixture is blended with the other components previously filled into aerosol containers.

The compositions of the invention can be employed as antiperspirants to reduce or eliminate perspiration as it appears at the skin surface.

According to a preferred method, the composition is applied to the skin, in particular where apocrine sweat glands are abundant, for example in the axillae, and in the genital and anal regions of the body, the film, layer or deposit of polymer so formed providing a means whereby perspiration is absorbed as it reaches the skin surface without the polymer becoming sticky when swollen with the moisture present in perspiration.

The invention is further illustrated by reference to the following Examples. The following words used in the Examples are Trade Marks: Bentone, National Starch, National Starch Resyn, Acrawax.

Example 1

This example illustrates a propellant based antiperspirant composition according to the invention.

The formulation of the composition was as follows:

| | % w/w |
|---|---|
| Cetyl alcohol (surfactant) | 1 |
| Bentone® 38 (suspending agent) | 0.6 |
| Isopropyl myristate (emollient) | 2 |
| Ethanol (solvent) | 0.3 |
| "Ammonium cross-linked starch" (polymer) | 3.5 |

| | % w/w |
|---|---|
| Sodium bicarbonate (deodorant) | 1.5 |
| Trichlorofluoromethan: Propellant 11 (solvent) | 45.55 |
| Monochlorodifluoromethane: Propellant 12 (solvent) | 45.55 |

In preparing this composition, the suspending agent was first milled with the propellant and this mixture was then blended with the other ingredients of the composition which had already been filled into aerosol containers. The containers were then fitted with conventional aerosol valves.

This composition when sprayed onto the axillae provided a sufficient protection against the appearance of perspiration for a period of at least 8 hours. The layer of polymer remained in close adherence to the skin and had a pleasant, smooth and unobtrusive feel. It was not noticeable when swollen with perspiration moisture and did not transfer to adjacent clothing.

Example 2

Example 1 was repeated using a formulation containing the following ingredients:

| | % w/w |
|---|---|
| Aluminium chlorhydrate | 1.5 |
| "Ammonium cross-linked starch" | 2.5 |
| Bentone® 38 | 0.36 |
| Cetyl alcohol | 0.5 |
| Isopropyl myristate | 1.8 |
| Ethanol | 0.18 |
| Perfume | 0.44 |
| Propellant 11/12 (50:50) | to 100 |

Example 3

Example 1 was repeated using a formulation containing the following ingredients:

| | % w/w |
|---|---|
| "Ammonium cross-linked starch" | 1 |
| Glycerol monolaurate (surfactant) | 1 |
| Bentone® 38 | 0.12 |
| Isopropyl myristate | 0.6 |
| Ethanol | 0.06 |
| Cetyl alcohol | 0.5 |
| Perfume | 0.44 |
| Propellant 11/12 (50:50) | to 100 |

Example 4

Example 1 was repeated using a formulation containing the following ingredients:

| | % w/w |
|---|---|
| "Ammonium cross-linked starch" | 3 |
| Isopropyl myristate | 5 |
| Ethanol | 20 |
| Bentone® 27 (suspending agent) | 1.5 |
| Cetyl alcohol | 0.5 |
| Propellant 11/12 (50:50) | to 100 |

Example 5

Example 1 was repeated using a formulation containing the following ingredients:

| | % w/w |
|---|---|
| "Sodium cross-linked starch" | 3 |
| Isopropyl myristate | 4 |
| Ethanol | 13.5 |
| Bentone® 27 | 1.5 |
| Cetyl alcohol | 1 |
| Propellant 11/12 (50:50) | to 100 |

Example 6

This example illustrates a formulation which is suitable for dispensing by means of a finger operated pump spray device.

The formulation of the composition was as follows:

| | % w/w |
|---|---|
| "Sodium cross-linked starch" | 15 |
| Industrial methylated spirit (solvent) | 70.12 |
| Isopropyl myristate | 11.9 |
| Bentone® 27 | 1.7 |
| Cetyl alcohol | 1.28 |

Example 7

Example 6 was repeated using the following formulation:

| | % w/w |
|---|---|
| "Ammonium cross-linked starch" | 15 |
| Industrial methylated spirit | 60.12 |
| Isopropyl myristate | 11.9 |
| Bentone® 27 | 1.7 |
| Cetyl alcohol | 1.28 |
| Water | 10 |

Example 8

Example 6 was repeated using the following formulation:

| | % w/w |
|---|---|
| National Starch Resyn® 78—3808 (polymer) | 7.5 |
| Industrial methylated spirit | 75.12 |
| Isopropyl myristate | 14.4 |
| Bentone® 27 | 1.7 |
| Cetyl alcohol | 1.28 |

Example 9

Example 6 was repeated using the following formulation:

| | % w/w |
|---|---|
| "Sodium cross-linked starch" polymer | 15 |
| Industrial methylated spirit | 70.12 |
| Isopropyl myristate | 11.9 |
| Bentone® 27 | 1.7 |
| Lauric acid monoglyceride (surfactant) | 1.28 |

Example 10

Example 6 was repeated using the following formulation:

| | % w/w |
|---|---|
| National Starch Resyn® 78—3803 | 7.5 |
| Industrial methylated spirit | 74 |
| Isopropyl myristate | 15 |
| Bentone® 38 | 0.5 |
| Cetyl alcohol | 0.5 |
| Zinc ricinoleate (suspending agent and deodorant) | 2.5 |

Example 11

Example 6 was repeated using the following formulation:

| | % w/w |
|---|---|
| "Sodium cross-linked starch" | 15 |
| Industrial methylated spirit | 70.12 |
| Isopropyl myristate | 11.9 |
| Cetyl alcohol | 1.28 |
| Acrawax® C (suspending agent) | 1.7 |

Example 12

This example illustrates a formulation which is suitable for dispensing from a roll-ball applicator.

The formulation of the composition was as follows:

| | % w/w |
|---|---|
| National Starch Resyn® 78—3803 | 7.5 |
| Industrial methylated spirit | 67.61 |
| Isopropyl myristate | 14.4 |
| Bentone® 27 | 1.7 |
| Cetyl alcohol | 1.28 |
| Water | 7.51 |

Example 13

Example 12 was repeated using the following formulation:

| | % w/w |
|---|---|
| National Starch Resyn® 78—3803 | 7.5 |
| Industrial methylated spirit | 60.1 |
| Isopropyl myristate | 14.4 |
| Bentone® 27 | 1.7 |
| Cetyl alcohol | 1.28 |
| Water | 15.02 |

Example 14

Example 12 was repeated using the following formulation:

| | % w/w |
|---|---|
| "Ammonium cross-linked starch" | 10 |
| Industrial methylated spirit | 71.25 |
| Isopropyl myristate | 15.0 |

| | % w/w |
|---|---|
| Bentone® 38 | 1.5 |
| Cetyl alcohol | 1.5 |
| Water | 0.5 |

Example 15

Example 12 was repeated using the following formulation:

| | % w/w |
|---|---|
| "Sodium cross-linked starch" | 15 |
| Industrial methylated spirit | 70 |
| Isopropyl myristate | 12 |
| Bentone® 38 | 0.5 |
| Cetyl alcohol | 1.0 |
| Zinc stearate (suspending agent) | 1.5 |

Example 16

Example 12 was repeated using the following formulation:

| | % w/w |
|---|---|
| National Starch Resyn® 78—3803 | 7.5 |
| Industrial methylated spirit | 74 |
| Isopropyl myristate | 15 |
| Bentone® 38 | 0.5 |
| Cetyl alcohol | 0.5 |
| Sodium stearate | 2.5 |

The antiperspirant compositions described herein and in particular in each of Examples 2 to 16, as well as that of Example 1, when applied to the skin, each provide a tenacious, skin substantive film which resists dislodgement by the user if the surface is rubbed, in that it does not flake or roll-up. The film also has a considerable capacity for absorbing skin moisture and remains dry and non-sticky and does not disintegrate when swollen with water present in skin moisture. The film furthermore has a pleasant silky smooth feel which is unobtrusive even when the polymer is swollen with perspiration moisture.

The film also has the ability to regenerate by losing absorbed moisture by evaporation so that it is again ready to absorb further skin moisture.

**Claims**

1. An antiperspirant composition having a solid phase and a liquid phase, the solid phase comprising a moisture-absorbent, water-insoluble polymer and the liquid phase comprising an organic solvent in which the said polymer is substantially insoluble and a surfactant, characterised in that

(i) the polymer amounts to from 0.1 to 30% by weight of the composition, is substantially dry to the touch when swollen with water and has a water-absorption value of at least 2,

(ii) the surfactant is selected from cetyl alcohol, isocetyl alcohol, stearyl alcohol, stearic acid, palmitic acid, glycerol monolaurate, and mixtures thereof, and amounts to from 0.1 to 15% by weight of the composition, and

(iii) the organic solvent amounts to from 1 to 99.8% by weight of the composition.

2. An antiperspirant composition according to Claim 1, characterised in that the polymer is a cross-linked starch.

3. An antiperspirant composition according to Claim 2, characterised in that the cross-linked starch is a cross-linked, gelatinised starch in which the degree of substitution of the cross-linking groups is 0.001 to 0.04.

4. An antiperspirant composition according to Claim 3, characterised in that the cross-linked gelatinised starch is substituted by ionic groups attached to the starch by ether linkages, which groups are associated with mono- or divalent counterions.

5. An antiperspirant composition according to any one of Claims 2 to 4, characterised in that the cross-linking is effected by ether bridges of the formula —O—R—O—, where R is an aliphatic group containing 1 to 10 carbon atoms.

6. An antiperspirant composition according to Claim 5, characterised in that R is $-CH_2CH(OH)CH_2-$.

7. An antiperspirant composition according to any one of Claims 2 to 6, characterised in that the ionic groups have the formula $Z-R^1-$, where $R^1$ is an alkylene group having 1 to 5 carbon atoms and Z is an anionic group selected from carboxyl, sulphonic or phosphonic groups or a cationic group of the formula

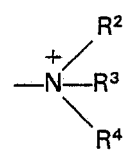

where $R^2$ is hydrogen or lower alkyl and $R^3$ and $R^4$ are lower alkyl or are alkylene groups linked together to form a five or six membered heterocyclic ring.

8. An antiperspirant composition according to any one of Claims 2 to 7, characterised in that the cross-linked starch is a carboxymethylated cross-linked starch.

9. An antiperspirant composition according

to any one of Claims 4 to 8, characterised in that the ionic groups are anionic groups and the counterions are alkali metal, alkaline earth metal, ammonium or substituted ammonium ions.

10. An antiperspirant composition according to Claim 9, characterised in that the counterion is sodium.

11. An antiperspirant composition according to Claim 1, characterised in that the polymer is an ionic complex of a water-soluble anionic polyelectrolyte and a polyvalent metal cation having a valency of at least three, the cation being present in the amount of 0.01 to 5.0 milliequivalents per gram of polyelectrolyte.

12. An antiperspirant composition according to Claim 11, characterised in that the polyelectrolyte contains anionic groups chosen from carboxylate, sulphonate, sulphate, phosphate and mixtures thereof.

13. An antiperspirant composition according to Claim 12, characterised in that the polyelectrolyte is polyacrylic acid.

14. An antiperspirant composition according to any one of Claims 11 to 13, characterised in that the polyvalent metal cation is aluminium.

15. An antiperspirant composition according to Claim 1, characterised in that the polymer is a covalently cross-linked anionic polyelectrolyte.

16. An antiperspirant composition according to Claim 15, characterised in that the anionic polyelectrolyte is a water-soluble anionic derivative of starch or cellulose, or a carboxylic homopolymer or copolymer containing at least 20 mole percent carboxylic units.

17. An antiperspirant composition according to Claim 16, characterised in that the carboxylic acid unit is polyacrylic acid.

18. An antiperspirant composition according to any one of Claims 15 to 17, characterised in that the covalent cross-linking compound is a divinyl compound or an allyl compound, a polyfunctional acrylate or methacrylate or a polyfunctional acrylamide or methacrylamide.

19. An antiperspirant composition according to any one of Claims 11 to 18, characterised in that the water-insoluble anionic polyelectrolyte complex has been surface treated with a polyvalent metal cation.

20. An antiperspirant composition according to Claim 19, characterised in that the polyvalent metal cation is aluminium.

21. An antiperspirant composition according to Claim 1, characterised in that the polymer is a base hydrolysed starch polyacrylonitrile graft copolymer.

22. An antiperspirant composition according to Claim 21, characterised in that the graft copolymer is one comprising water-insoluble alkali metal salts of saponified gelatinised starch and saponified polyacrylonitrile in mole ratios of from 1:1.5 to 1:9.

23. An antiperspirant composition according to any preceding claim, characterised in that the polymer has a water absorption value of from 5 to 500.

24. An antiperspirant composition according to any preceding claims, characterised in that the organic solvent is a propellant.

25. An antiperspirant composition according to any one of Claims 1 to 23, characterised in that the organic solvent is chosen from ethyl alcohol, methylene chloride, isopropyl alcohol, ethylene glycol monomethyl ether, pentane, hexane, heptane, octane, linear and cyclic polydimethyl siloxane and mixtures thereof.

26. An antiperspirant composition according to any preceding claim, characterised in that it further comprises an emollient.

27. An antiperspirant composition according to any preceding claim, characterised in that it further comprises a suspending agent.

28. An antiperspirant composition according to any preceding claim, characterised in that it further comprises a deodorant.

29. An antiperspirant composition according to Claim 27 or Claim 28, characterised in that it comprises as suspending agent and/or deodorant zinc ricinoleate.

**Patentansprüche**

1. Ein Schweißverhütungsmittel mit einer festen Phase und einer flüssigen Phase, wobei die feste Phase ein feuchtigkeitsabsorbierendes wasserunlösliches Polymer und die flüssige Phase ein organisches Lösungsmittel, worin das Polymer praktisch unlöslich ist, und ein Tensid umfaßt, dadurch gekennzeichnet, daß

(i) das Polymer 0,1 bis 30 Gewichtsprozent des Mittels ausmacht, mit Wasser gequollen bei Berührung praktisch trocken ist und einen Wasserabsorptionswert von wenigstens 2 hat,

(ii) das Tensid unter Cetylalkohol, Isocetylalkohol, Stearylalkohol, Stearinsäure, Palmitinsäure, Glycerinmonolaurat und deren Gemischen ausgewählt ist und 0,1 bis 15 Gewichtsprozent des Mittels ausmacht, und

(iii) das organische Lösungsmittel 1 bis 99,8 Gewichtsprozent des Mittels ausmacht.

2. Schweißverhütungsmittel nach Anspruch 1, dadurch gekennzeichnet, daß das Polymer eine vernetzte Stärke ist.

3. Schweißverhütungsmittel nach Anspruch 2, dadurch gekennzeichnet, daß die vernetzte Stärke eine vernetzte gelatinierte Stärke ist, in der der Subsitutionsgrad der vernetzenden Gruppen 0,001 bis 0,04 ist.

4. Schweißverhütungsmittel nach Anspruch 3, dadurch gekennzeichnet, daß die vernetzte gelatinierte Stärke durch ionische Gruppen, die an der Stärke über Ätherbindungen hängen und mit ein- oder zweiwertigen Gegenionen assoziiert sind, substituiert ist.

5. Schweißverhütungsmittel nach einem der

Ansprüche 2 bis 4, dadurch gekennzeichnet, daß die Vernetzung durch Ätherbrücken der formel —O—R—O— erfolgt ist, wobei R eine aliphatische Gruppe mit 1 bis 10 Kohlenstoffatomen ist.

6. Schweißverhütungsmittel nach Anspruch 5, dadurch gekennzeichnet, daß R $-CH_2CH(OH)CH_2-$ ist.

7. Schweißverhütungsmittel nach einem der Ansprüche 2 bis 6, dadurch gekennzeichnet, daß die ionischen Gruppen die Formel Z—$R^1$— haben, worin $R^1$ eine Alkylengruppe mit 1 bis 5 Kohlenstoffatomen und Z eine anionische Gruppe, ausgewählt unter Carboxyl, Sulfon- oder Phosphongruppen, oder eine kationische Gruppe der Formel

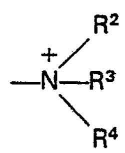

ist, worin $R^2$ Wasserstoff oder Niederalkyl und $R^3$ und $R^4$ Niederalkyl oder miteinander zu einem 5- oder 6-gliedrigen heterocyclischen Ring verbundene Alkylengruppen sind.

8. Schweißverhütungsmittel nach einem der Ansprüche 2 bis 7, dadurch gekennzeichnet, daß die vernetzte Stärke eine carboxymethylierte vernetzte Stärke ist.

9. Schweißverhütungsmittel nach einem der Ansprüche 4 bis 8, dadurch gekennzeichnet, daß die ionischen Gruppen anionische Gruppen und die Gegenionen Alkalimetall-, Erdalkalimetall-, Ammonium- oder substituierte Ammoniumionen sind.

10. Schweißverhütungsmittel nach Anspruch 9, dadurch gekennzeichnet, daß das Gegenion Natrium ist.

11. Schweißverhütungsmittel nach Anspruch 1, dadurch gekennzeichnet, daß das Polymer ein ionischer Komplex eines wasserlöslichen anionischen Polyelektrolyten und eines mehrwertigen Metallkations mit einer Wertigkeit von wenigstens drei ist, das in einer Menge von 0,01 bis 5,0 Milliäquivalente pro Gramme Polyelektrolyt vorliegt.

12. Schweißverhütungsmittel nach Anspruch 11, dadurch gekennzeichnet, daß der Polyelektrolyt anionische Gruppen, ausgewählt unter Carboxylat, Sulfonat, Sulfat, Phosphat und deren Gemischen, enthält.

13. Schweißverhütungsmittel nach Anspruch 12, dadurch gekennzeichnet, daß der Polyelektrolyt Polyacrylsäure ist.

14. Schweißverhütungsmittel nach einem der Ansprüche 11 bis 13, dadurch gekennzeichnet, daß das mehrwertige Metall-Kation Aluminium ist.

15. Schweißverhütungsmittel nach Anspruch 1, dadurch gekennzeichnet, daß das Polymer ein kovalent vernetzter anionischer Polyelektrolyt ist.

16. Schweißverhütungsmittel nach Anspruch 15, dadurch gekennzeichnet, daß der anionische Polyelektrolyt ein wasserlösliches anionisches Derivat von Stärke oder Cellulose oder ein carboxylisches Homopolymer oder Copolymer, das wenigstens 20 Mol-% Carboxyleinheiten enthält, ist.

17. Schweißverhütungsmittel nach Anspruch 16, dadurch gekennzeichnet, daß die Carbonsäure-Einheit Polyacrylsäure ist.

18. Schweißverhütungsmittel nach einem der Ansprüche 15—17, dadurch gekennzeichnet, daß die kovalente vernetzende Verbindung eine Divinylverbindung oder eine Allylverbindung, ein polyfunktionelles Acrylat oder Methacrylat oder ein polyfunktionelles Acrylamid oder Methacrylamid ist.

19. Schweißverhütungsmittel nach einem der Ansprüche 11 bis 18, dadurch gekennzeichnet, daß der wasserunlösliche anionische Polyelektrolytkomplex mit einem mehrwertigen Metallkation oberflächenbehandelt worden ist.

20. Schweißverhütungsmittel nach Anspruch 19, dadurch gekennzeichnet, daß das mehrwertige Metallkation Aluminium ist.

21. Schweißverhütungsmittel nach Anspruch 1, dadurch gekennzeichnet, daß das Polymer ein basenhydrolysierte Stärke/Polyacrylnitril-Pfropfcopolymerisat ist.

22. Schweißhemmendes Mittel nach Anspruch 21, dadurch gekennzeichnet, daß das Pfropfcopolymerisat ein wasserunlösliche Alkalimetallsalze verseifter, gelatinierter Stärke und verseiften Polyacrylnitrils in Molverhältnissen von 1:1,5 bis 1:9 aufweisendes ist.

23. Schweißhemmendes Mittel nach irgend einem vorhergehenden Anspruch, dadurch gekennzeichnet, daß das Polymer einen Wasserabsorptionswert von 5 bis 500 hat.

24. Schweißhemmendes Mittel nach einem der vorhergehenden Ansprüche, dadurch gekennzeichnet, daß das organische Lösungsmittel ein Treibmittel ist.

25. Schweißverhütungsmittel nach einem der Ansprüche 1 bis 23, dadurch gekennzeichnet, daß das organische Lösungsmittel unter Ethylalkohol, Methylenchlorid, Isopropylalkohol, Ethylenglykol-monomethylether, Pentan, Hexan, Heptan, Octan, linearem und cyclischem Polydimethylsiloxan und deren Gemischen ausgewählt ist.

26. Schweißverhütungsmittel nach irgend einem vorhergehenden Anspruch, dadurch gekennzeichnet, daß es ferner ein weichmachendes Mittel umfaßt.

27. Schweißverhütungsmittel nach irgend einem vorhergehenden Anspruch, dadurch gekennzeichnet, daß es ferner ein suspendierendes Mittel umfaßt.

28. Schweißverhütungsmittel nach irgend einem vorhergehenden Anspruch, dadurch gekennzeichnet, daß es ferner ein Desodorierungsmittel umfaßt.

29. Schweißverhütungsmittel nach Anspruch 27 oder 28, dadurch gekennzeichnet, daß es als suspendierendes und/oder desodoriendes Mittel Zinkricinoleat umfaßt.

**Revendications**

1. Composition anti-transpirante ayant une phase solide et une phase liquide, la phase solide comprenant un polymère absorbant l'humidité, insoluble dans l'eau et la phase liquide comprenant un solvant organique dans lequel ledit polymère est pratiquement insoluble et un agent tensioactif, caractérisée en ce que
(i) le polymère s'élève à 0,1 à 30% en poids de la composition, est pratiquement sec au toucher lorsqu'il est gonflé d'eau et possède une valeur d'absorption d'eau d'au moins 2,
(ii) l'agent tensioactif est choisi parmi l'alcool cétylique, l'alcool isocétylique, l'alcool stéarylique, l'acide stéarique, l'acide palmitique, le monolaurate de glycérol, et leurs mélanges, et s'élève à 0,1 à 15% en poids de la composition, et
(iii) le solvant organique s'élève à 1 à 99,8% en poids de la composition.

2. Composition anti-transpirante selon la revendication 1, caractérisée en ce que le polymère est unamidon réticulé.

3. Composition anti-transpirante selon la revendication 2, caractérisée en ce que l'amidon réticulé est un amidon réticulé, gélatinisé dans lequel le degré de substitution des groupes réticulants est de 0,001 à 0,04.

4. Composition anti-transpirante selon la revendication 3, caractérisée en ce que l'amidon gélatinisé réticulé est substitué par des groupes ioniques attachés à l'amidon par des liaisons éther, groupes qui sont associés avec des ions complémentaires mono- ou di-valents.

5. Composition anti-transpirante selon l'une quelconque des revendications 2 à 4, caractérisée en ce que la réticulation est effectuée par des ponts éther de formule —O—R—O, où R est un groupe aliphatique contenant de 1 à 10 atomes de carbone.

6. Composition anti-transpirante selon la revendication 5, caractérisée en ce que R est $—CH_2CH(OH)CH_2—$.

7. Composition anti-transpirante selon l'une quelconque des revendications 2 à 6, caractérisée en ce que les groupes ioniques ont la formule $Z—R^1—$, où $R^1$ est un groupe alcoylène ayant de 1 à 5 atomes de carbone et Z est un groupe anionique choisi entre les groupes carboxyle, sulfonique ou phosphonique ou un groupe cationique de formule

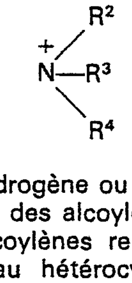

où $R^2$ est un hydrogène ou un alcoyle inférieur et $R^3$ et $R^4$ sont des alcoyle inférieurs ou sont des groupes alcoylènes reliés ensemble pour former un noyau hétérocyclique à 5 ou 6 chaînons.

8. Composition anti-transpirante selon l'une quelconque des revendications 2 à 7, caractérisée en ce que l'amidon réticulé est un amidon réticulé carboxyméthylé.

9. Composition anti-transpirante selon l'une quelconque des revendications 4 à 8, caractérisée en ce que les groupes ioniques sont des groupes anioniques et en ce que les ions complémentaires sont des ions métal alcalin, métal alcalinoterreux, ammonium ou ammonium substitué.

10. Composition anti-transpirante selon la revendication 9, caractérisé en ce que l'ion complémentaire est le sodium.

11. Composition anti-transpirante selon la revendication 1, caractérisée en ce que le polymère est un complexe ionique d'un polyélectrolyte anionique soluble dans l'eau et d'un cation métallique polyvalent ayant une valence d'au moins 3, le cation étant présent en une quantité de 0,01 à 5,0 milliéquivalents par g de polyélectrolyte.

12. Composition anti-transpirante selon la revendication 11, caractérisée en ce que le polyélectrolyte contient des groupes anioniques choisis parmi le carboxylate, le sulfonate, le sulfate, le phosphate et leurs mélanges.

13. Composition anti-transpirante selon la revendication 12, caractérisée en ce que le polyélectrolyte est l'acide polyacrylique.

14. Composition anti-transpirante selon l'une quelconque des revendications 11 à 13, caractérisée en ce que le cation métallique polyvalent est l'aluminium.

15. Composition anti-transpirante selon la revendication 1, caractérisée en ce que le polymère est un polyélectrolyte anionique réticulé de façon covalente.

16. Composition anti-transpirante selon la revendication 15, caractérisée en ce que le polyélectrolyte anionique est un dérivé anionique soluble dans l'eau de l'amidon ou de la cellulose, ou un homopolymère ou copolymère carboxylique contenant au moins 20% molaire d'unités carboxyliques.

17. Composition anti-transpirante selon la revendication 16, caractérisée en ce que l'unité acide carboxylique est l'acide polyacrylique.

18. Composition anti-transpirante selon l'une quelconque des revendications 15 à 17, caractérisée en ce que le composé réticulant covalent est un composé divinylique ou un composé allylique, un acrylate ou méthacrylate polyfonctionnel ou un acrylamide ou méthacrylamide polyfonctionnel.

19. Composition anti-transpirante selon l'une quelconque des revendications 11 à 18, caractérisée en ce que le complexe polyélectrolytique anionique insoluble dans l'eau a été traité à sa surface avec un cation métallique polyvalent.

20. Composition anti-transpirante selon la revendication 19, caractérisée en ce que le cation métallique polyvalent est l'aluminium.

21. Composition anti-transpirante selon la revendication 1, caractérisée en ce que le polymère est un copolymère greffé amidon hydrolysé par une base/polyacrylonitrile.

22. Composition anti-transpirante selon la revendication 21, caractérisée en ce que le copolymère greffé est un copolymère comprenant des sels de métaux alcalins insolubles dans l'eau d'amidon gélatinisé saponifié et de polyacrylonitrile saponifié dans des rapports molaires allant de 1:1,5 à 1:9.

23. Composition anti-transpirante selon l'une quelconque des revendications précédentes, caractérisé en ce que le polymère a une valeur d'absorption d'eau allant de 5 à 500.

24. Composition anti-transpirante selon l'une quelconque des revendications précédentes, caractérisée en ce que le solvant organique est un agent propulseur.

25. Composition anti-transpirante selon l'une quelconque des revendications 1 à 23, caractérisée en ce que le solvant organique est choisi parmi l'alcool éthylique, le chlorure de méthyléne, l'alcoolisopropylique, le monométhyléther d'éthylèneglycol, le pentane, l'hexane, l'heptane, l'octane, le polydiméthylsiloxane linéaire et cyclique et leurs mélanges.

26. Composition anti-transpirante selon l'une quelconque des revendications précédentes, caractérisée en ce qu'elle contient en outre un émollient.

27. Composition anti-transpirante selon l'une quelconque des revendications précédentes, caractérisée en ce qu'elle contient en outre un agent suspenseur.

28. Composition anti-transpirante selon l'une quelconque des revendications précédentes, caractérisée en ce qu'elle contient en outre un désodorant.

29. Composition anti-transpirante selon la revendication 27 ou la revendication 28, caractérisée en ce qu'elle comprend comme agent suspenseur et/ou désodorant le ricinoléate de zinc.